# EUROPÄISCHE PATENTSCHRIFT

(11) **EP 0 653 422 B1**
(45) Veröffentlichungstag und Bekanntmachung des Hinweises auf die Patenterteilung: **25.02.1998**
(21) Anmeldenummer: 94117951.7
(22) Anmeldetag: 14.11.1994
(51) Int. Cl.: C07D 233/68, C07D 233/96

(54) **Verfahren zur Herstellung von 2-substituierten 5-Chlorimidazol-4-carbaldehyden**
Process for the preparation of 2-substituted 5-chlorimidazole-4-carbaldehydes
Procédé pour la préparation de 5-chloro-imidazole-4-carbaldéhydes substituées en 2

(30) Priorität: 15.11.1993 CH 3410/93
(43) Veröffentlichungstag der Anmeldung: 17.05.1995
(73) Patentinhaber: LONZA AG, CH-3945 Gampel/Wallis (CH)
(72) Erfinder: Griffiths, Dr. Gareth, CH-3930 Visp, (Kanton Wallis) (CH); Gosteli, Dr. Jacques, CH-4059 Basel, (Kanton Wallis) (CH); Imwinkelried, Dr. René, CH-3902 Brig-Glis, (Kanton Wallis) (CH)
(74) Vertreter: Weinhold, Peter, Dr.

(56) Entgegenhaltungen:
- EP-A- 0 028 834

## Beschreibung

Die Erfindung beinhaltet ein neues Verfahren zur Herstellung von 2-substituierten 5-Chlorimidazol-4-carbaldehyden der allgemeinen Formel 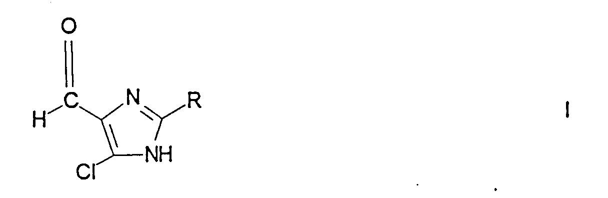 worin R Wasserstoff, eine Alkylgruppe, eine Alkenylgruppe, eine Cycloalkylgruppe, eine Arylalkylgruppe oder eine Arylgruppe bedeutet.

Die 2-substituierten 5-Chlorimidazol-4-carbaldehyde der allgemeinen Formel I sind wichtige Ausgangsprodukte Zur Herstellung von blutdrucksenkenden Pharmazeutika (US-PS 4 355 040) oder von herbizidwirksamen Verbindungen (DE-OS 2804435).

Es sind mehrere Wege zur Herstellung der genannten Verbindungen gemäss allgemeiner Formel I bekannt.

So beschreibt die US-PS 4 355 040 ein Verfahren, nach welchem 2-Amino-3,3-dichloracrylnitril mit einem Aldehyd zum entsprechenden Azomethinzwischenprodukt und weiter mit einem Halogenwasserstoff und Wasser zum 2-substituierten-5-Halogen-imidazol-4-carbaldehyd umgesetzt wird. Experimentielle Angaben fehlen in der genannten Patentschrift. Ein grosser Nachteil der Synthese besteht darin, dass das eingesetzte 2-Amino-3,3-dichloracrylnitril zunächst ausgehend von Dichloracetonitril durch dessen Umsetzung mit Blausäure/Natriumcyanid hergestellt werden muss. Die äusserst toxischen Reaktionsteilnehmer und die damit verbundenen sicherheitstechnischen Massnahmen, die bereits für die Bereitstellung des Ausgangsproduktes notwendig sind, machen das Gesamtverfahren industriell untauglich.

Die US-PS 4 355 040 offenbart in einer weiteren Variante ein 3-Stufen-Verfahren, worin in einer ersten Stufe ein Amidin-hydrochlorid mit Dihydroxyaceton mit hohem NH₃-Druck ringgeschlossen wird, der Imidazolalkohol halogeniert wird und schliesslich Zum Aldehyd oxidiert wird.
Es hat sich gezeigt, dass für die Ringschlussreaktion Drucke von über 20 bar notwendig sind. Die Oxidation des Alkohols funktioniert ausserdem in Gegenwart von Chromoxid. Es ist offensichtlich, dass eine Oxidation mit Schwermetalloxiden, die in der Regel nicht reztrkulierbar sind, nach heutigen ökologischen Gesichtspunkten nicht mehr verantwortbar ist.

Es bestand folglich die Aufgabe, ein Verfahren zu entwickeln, das die genannten Nachteile nicht aufweist.

Die Aufgabe konnte gelöst werden mit einem neuen Verfahren nach Anspruch 1.

Die allgemeinen Bezeichnungen der Gruppen in den Substituenten R, R₁, R₂, R₃, R₄, R₅ und R₆ in den allgemeinen Formeln I bis VI haben nachfolgende Bedeutung.

Unter einer Alkylgruppe wird eine geradkettige oder verzweigte (C₁-C₆)-Alkylgruppe, insbesondere Methyl, Ethyl, n-Propyl, i-Propyl, n-Butyl, sec. Butyl, tert. Butyl, Pentyl und seine Isomeren oder Hexyl und seine Isomeren verstanden. Bevorzugte Alkylgruppe für R ist die n-Butylgruppe. Bevorzugte Alkylgruppe für R₁, R₂, R₃, R₄, R₅ und R₆ ist eine (C₁-C₄)-Alkylgruppe.
Unter einer Alkenylgruppe wird eine geradkettige oder verzweigte (C₂-C₆)-Alkenylgruppe, wie z. B. 1-Propenyl, 2-Propenyl, 1-Butenyl, 2-Butenyl, 3-Butenyl, Pentenyl und seine Isomeren oder Hexenyl und seine Isomeren,verstanden. Bevorzugte Alkenylgruppe ist die 2- oder 3-Butenylgruppe.

Als Vertreter von Cycloalkylgruppen seien die Cyclopropyl-, die Cyclobutyl-, die Cyclopentyloder die Cyclohexylgruppe genannt.

Unter einer Arylalkylgruppe wird zweckmässig eine Phenyl-(C₁-C₆)alkyl-, bevorzugt eine Berzylgruppe,verstanden.
Aryl hat zweckmässig die Bedeutung von Phenyl.

Sowohl die Arylalkylgruppe als auch die Arylgruppe kann an ihrem aromatischen Kern einen oder mehrere Substituenten wie z. B. Alkyl, Halogen, Nitro oder Amino aufweisen.

Unter der Bezeichnung Halogen wird zweckmässig Chlor, Brom oder Jod,vorzugsweise Chlor, verstanden.

Im ersten Schritt des erfindungsgemässen Verfahrens wird ein Glycinesterhydrohalogenid der allgemeinen Formel 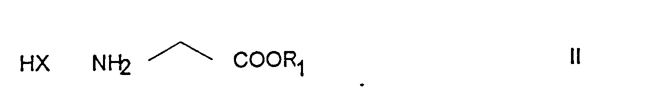 worin R₁ eine Akylgruppe bedeutet und X für ein Halogenatom steht,mit einem Imidsäureester der allgemeinen Formel 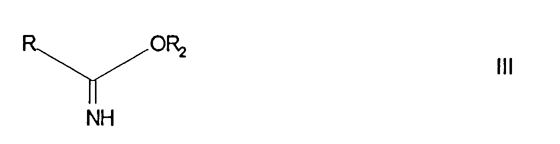 worin R die genannte Bedeutung hat und R₂ eine Alkylgruppe bedeutet,in Gegenwart einer Base zum 2-substituierten 3,5-Dihydroimidazol-4-on der allgemeinen Formel 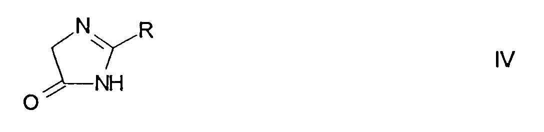 worin R die genannte Bedeutung hat, umgesetzt.

Zweckmässig wird so vorgegangen, dass das Glycinesterhydrohalogenid der allgemeinen Formel II in Gegenwart einer Base, zweckmässig bei einem pH-Wert von 7 bis 12, vorzugsweise von 9 bis 11,mit dem Imidsäureester der allgemeinen Formel III reagiert wird.
Die Glycinesterhydrohalogenide der allgemeinen Formel II sind im Handel erhältliche stabile Verbindungen.
Geeignete Basen sind die Alkalihydroxide, wie z. B. Natriumhydroxid oder Kaliumhydroxid, oder Alkalialkoholate wie z. B. Natrium- oder Kaliummethylat, -ethylat oder -tert.butylat.

Vorteilhaft liegt die Base in einem geeigneten Lösungsmittel gelöst vor. Besonders geeignet sind aliphatische Alkohole, wie Methanol oder Ethanol. Der Imidsäureester wird zweckmässig ebenfalls in Form einer Lösung in einem inerten Lösungsmittel zugegeben. In der Regel eignen sich hierzu aromatische Lösungsmittel, wie z..B. Toluol oder Chlorbenzol,oder die genannten aliphatischen Alkohole besonders gut.

Von Vorteil erfolgt die Umsetzung der Reaktionsteilnehmer Glycinesterhydrohalogenid, Imidsäureester und Base im stöchiometrischen Verhältnis 1:1:1.

Die Umsetzungstemperatur rangiert zweckmässig im Bereich von -20° C bis 50° C, vorzugsweise bei 0° bis 25° C.

Nach einer Umsetzungszeit von wenigen Stunden kann das entsprechende 2-substituierte 3,5-Dihydroimidazol-4-on der allgemeinen Formel IV auf fachmännische Weise , in der Regel durch einfache Filtration,in Ausbeuten von grösser als 95% isoliert werden.

Von Vorteil wird jedoch das genannte Imidazolinon der allgemeinen Formel IV nicht isoliert, sondern dem resultierenden Reaktionsgemisch wird direkt der Reaktand der zweiten Stufe, das N,N-subst. Formamidacetal der allgemeinen Formel 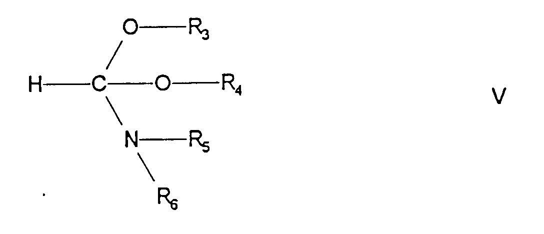 worin R₃ und R₄ gleich oder verschieden sind und eine Alkylgruppe oder eine Arylalkylgruppe bedeuten und R₅ und R₆ gleich oder verschieden sind und für eine Alkylgruppe, eine Arylalkylgruppe oder eine Arylgruppe stehen, zugegeben.

Geeignete N,N-subst. Formamidacetale der allgemeinen Formel V sind die N,N-Dimethylformamiddialkylacetale. Besonders bevorzugt ist das N,N-Dimethylformamiddimethylacetal, worin R₃, R₄, R₅ und R₆ Methyl bedeuten.

Die Umsetzung in der zweiten Stufe kann in Gegenwart eines inerten Lösungsmittels wie z. B. in einem aliphatischen Alkohol, einem halogenierten Kohlenwasserstoff oder einem Aromaten erfolgen. Mit guten Ergebnissen konnten daher Methanol, Methylenchlorid oder Toluol verwendet werden. Es ist aber auch möglich,ohne zusätzliches Lösungsmittel, d. h. in Gegenwart des Acetals als Lösungsmittel,zu arbeiten.
Die Reaktion verläuft zweckmässig bei einer Temperatur zwischen -50°C und 100°C,bevorzugt aber bei Raumtemperatur.

Das resultierende N,N-subst.-Aminomethylenimidazolinon der allgemeinen Formel 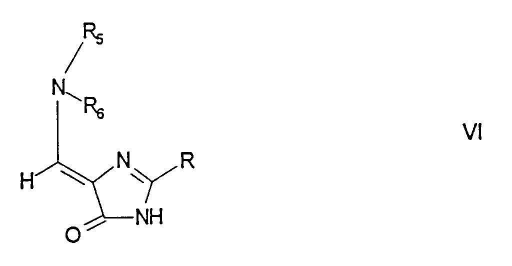 worin R, R₅ und R₆ die genannte Bedeutung haben, ist nicht literaturbekannt und als wichtiges Zwischenprodukt in der erfindungsgemässen Synthese ebenfalls Bestandteil der Erfindung.

Das genannte N,N-subst. Aminomethylenimidazolinon kann als E- oder Z-Isomer auftreten.

Besonders bevorzugtes Imidazolinon der allgemeinen Formel VI ist das (Z)-2-Butylderivat mit R = n-Butyl und R₅, R₆ = Methyl.

Das N,N-subst. Aminomethylenimidazolinon der allgemeinen Formel VI kann auf fachmännisch übliche Weise aus dem Reaktionsgemisch isoliert werden.

Es ist aber ebenso möglich,ohne Isolation dieses Zwischenproduktes die Chlorierung mit Phosphoroxychlorid oder Phosgen in der dritten und letzten Stufe anzuschliessen.

Die Chlorierung kann dabei in Gegenwart von Phosphoroxychlorid alleine oder in Gegenwart des sogenannten Vilsmeier-Reagenz, welches aus Phosphoroxychlorid und N,N-Dimethylformamid oder Phosgen und N,N-Dimethylformamid, zweckmässig im Molverhältnis 1 zu 1 bis 4 zu 1,besteht, erfolgen.

Die genannten Chlorierungsmittel werden zweckmässig im Überschuss eingesetzt und dienen somit gleichzeitig als Lösungsmittel.

Allerdings ist es auch möglich,in Gegenwart eines zusätzlichen inerten Lösungsmittels zu arbeiten.

Die Chlorierung wird zweckmässig bei einer Temperatur zwischen 50°C und 150°C durchgeführt.
Nach einer Reaktionszeit von ca. 0.5 h bis 4 h kann der entsprechende 2-subst.-5-Chlorimidazol-4-carbaldehyd der allgemeinen Formel I auf fachmännische Weise, zweckmässig durch Behandlung des Reaktionsgemisches mit Wasser und Extraktion mit einem geeigneten Lösungsmittel,in guter Ausbeute und Reinheit erhalten werden.

### Beispiel 1

### Herstellung von (Z)-2-Butyl-4-dimethylaminomethylen-2-imidazolin-5-on VI aus 2-Butyl-2-imidazol-5-on IV

Zu einer Lösung von 2 Butyl-2-imidazol-5-on (2,80 g, 20 mmol) in Methanol (20 ml) wurde N,N-Dimethylformamid-dimethylacetal (2,85 g, Gehalt ca 92%, 22 mmol) zugegeben (die Temperatur stieg von 18°C auf 26°C). Nach 45 Minuten wurde die Lösung eingeengt und am Hochvakuum getrocknet. Es wurden 3,88 g 2-Butyl-4-dimethylaminomethylen-2-imidazolin-5-on erhalten mit einem Gehalt von >90% nach H-NMR. Dies entspricht einer Ausbeute von ca. 90%,bez. auf 2-Butyl-2-imidazol-5-on. Das Produkt konnte aus Essigester umkristallisiert werden.
- Smp.:: 114 - 116,5°C
- ¹H-NMR (CDCl₃, 400 MHz): 0,95 (3H, t);
1,42 (2H, m);
1,68 (2H, m);
2,53 (2H, t);
3,17 (3H, br s);
3,55 (3H, br s);
7,03 (1H, s);
10,35 (1H, br s).

### Beispiel 2

### Herstellung von (Z)-2-Butyl-4-dimethylaminomethylen-2-imidazolin-5-on VI aus Pentanimidsäure-methylester III

Zu einer Lösung von Natriumhydroxid (1,59 g, 39,42 mmol) in Methanol (13 ml) bei 0°C wurde Glycinmethylester-hydrochlorid (5,00 g, 39,42 mmol) in einer Portion zugegeben, wobei die Temperatur auf -10°C sank. Das Gemisch wurde 15 Minuten gerührt, während dieser Zeit stieg die Temperatur wieder auf 0°C. Pentanimidsäuremethylester (4,73 g, 39,42 mmol) wurde zugegeben,und das Gemisch wurde 3 Stunden bei Raumtemperatur gerührt. Danach wurde N,N-Dimethylformamid-dimethylacetal (5,62 g, 43,39 mmol) während 5 Minuten zugegeben,und das Reaktionsgemisch wurde weitere 3 Stunden gerührt. Das Lösungsmittel wurde am Rotavapor entfernt,und der Rückstand wurde mit CH₂Cl₂ (40 ml) und Wasser (15 ml) versetzt.Nach Phasentrennung wurde die organische Phase mit Wasser (10 ml) gewaschen, anschliessend wurden die vereinigten H₂O-Phasen zweimal mit je 20 ml CH₂Cl₂ gewaschen. Die vereinigten organischen Phasen wurden getrocknet (MgSO₄), filtriert, am Rotavapor eingeengt und am Hochvakuum getrocknet.

Es wurden 6,70 g 2-Butyl-4-dimethylaminomethylen-2-imidazolin-5-on erhalten mit einem Gehalt von ca. 90% nach ¹H-NMR. Dies entspricht einer Ausbeute von ca 78%,bez. auf Pentanimidsäuremethylester.

### Beispiel 3

### Herstellung von 2-Butyl-5-chlorimidazol-4-carbaldehyd I aus (Z)-2-Butyl-4-dimethylaminomethylen-2-imidazolin-5-on IV

Ein Gemisch von 2-Butyl-4-dimethylaminomethylen-2-imidazolin-5-on (1,00g,5, 12 mmol) und POCl₃ (3,20 g, 20,48 mmol) wurde während 45 Minuten bei 100°C erhitzt. 1,76 g POCl₃ wurden am Rotavapor abdestilliert,und der Rückstand wurde mit Essigsäureethylester (6 ml) versetzt. Das so erhaltene Gemisch wurde zu Wasser (20 ml) zugegeben und 5 Minuten bei Raumtemperatur gerührt. Der pH wurde mit 30%iger Natronlauge von 0,34 auf 7 gestellt,und das Gemisch wurde zweimal mit je 10 ml Essigsäureethylester extrahiert. Die vereinigten organischen Phasen wurden getrocknet (MgSO₄), filtriert, eingeengt und am Hochvakuum getrocknet.
Es wurden 0,89 g 2-Butyl-5-chlorimidazol-4-carbaldehyd (Reinheit >95% nach H-NMR) erhalten. Dies entspricht einer Ausbeute von 93%,bez. auf 2-Butyl-4-dimethylaminomethylen-2-imidazolin-5-on.

### Beispiel 4

### Herstellung von (Z)- 2-Butyl-4-dimethylaminomethylen-2-imidazolidin-5-on VI aus 2-Butyl-2-imidazolin-5-on IV

Eine Lösung von 2-Butyl-2-imidazolin-5-on (5,00 g, 35,67 mmol) und N,N-Dimethylformamid-diisopropylacetal (7,02 g, 39,24 mmol) in Methylenchlorid (25 ml) wurde 2,5 h bei Raumtemperatur gerührt. Das Lösungsmittel wurde am Rotavapor entfernt,und der Rückstand wurde mit Methylenchlorid (40 ml) versetzt. Die so erhaltene Lösung wurde zweimal mit je 10 ml Wasser gewaschen, getrocknet (MgSO₄), am Rotavapor eingeengt und am Hochvakuum getrocknet. Es wurden 5,37 g 2-Butyl-4-dimethylaminomethylen-2-imidazolin-5-on erhalten mit einem Gehalt von >90% nach H-NMR. Dies entspricht einer Ausbeute von ca. 71%,bez. auf 2-Butyl-2-imidazolin-5-on.

### Beispiel 5

### Herstellung von (Z)- 2-Butyl-4-dimethylaminomethylen-2-imidazolin-5-on VI aus Pentanimidsäuremethylester III

Zu einer Losung von Natriumhydroxid (1,59 g, 39,42 mmol) in Methanol (13 ml) bei 0°C wurde Glycinmethylester-hydrochlorid (5,00 g, 39,42 mmol) in einer Portion zugegeben, wobei die Temperatur auf -10°C sank. Das Gemisch wurde 15 Minuten gerührt, während dieser Zeit stieg die Temperatur wieder auf 0°C. Pentanimidsäuremethylester (4,73 g, 39,42 mmol) wurde zugegeben,und das Gemisch wurde 3 h bei Raumtemperatur gerührt. Danach wurde N,N-Dimethylformamid-dibutylacetal (9,00 g, 43,36 mmol) während 5 Minuten zugegeben,und das Reaktionsgemisch wurde weitere 3 h gerührt. Das Lösungsmittel wurde am Rotavapor entfernt,und der Rückstand wurde mit CH₂Cl₂ (40 ml) und Wasser (15 ml) versetzt. Nach Phasentrennung wurde die organische Phase mit Wasser (10 ml) gewaschen. Die organische Phase wurde getrocknet (MgSO₄), filtriert, am Rotavapor eingeengt und am Hochvakuum getrocknet.
Es wurden 6,92 g 2-Butyl-4-dimethylaminomethylen-2-imidazolin-5-on erhalten mit einem Gehalt von ca. 80% nach H-NMR. Dies entspricht einer Ausbeute von ca. 72%,bez. auf Pentanimidsäuremethylester.

### Beispiel 6

### Herstellung von 2-Butyl-5-chlorimidazol-4-carbaldehyd I aus Pentanimidsäuremethylester III

Zu einer Lösung von Natriumhydroxid (10,13 g, 250 mmol) in Methanol (80 ml) bei 0°C wurde Glycinmethylester-hydrochlorid (31,72 g, 250 mmol) in einer Portion zugegeben, wobei die Temperatur auf -10°C sank. Das Gemisch wurde 10 Minuten gerührt, während dieser Zeit stieg die Temperatur wieder auf 0°C. Pentanimidsäuremethylester (108,25 g einer 26,6%-igen Lösung in Toluol, 250 mmol) wurde zugegeben,und das Gemisch wurde 3 h bei Raumtemperatur gerührt. Danach wurde N,N-Dimethylformamid-dimethylacetal (35,64 g, ca. 92%-ig, 275 mmol) während 5 Minuten zugegeben,und das Reaktionsgemisch wurde weitere 3 h gerührt. Toluol (200 ml) wurde zugegeben,und Methanol und Wasser wurden durch Destillation unter Vakuum aus dem Gemisch entfernt. Von den verbleibenden 203,5 g wurden 91,68 g (entsprechend 112 mmol Pentanimidsäuremethylester) bei Raumtemperatur vorgelegt und mit POCl₃ (65,09 g, 416 mmol) versetzt. Das Gemisch wurde 1,5 h bei 100°C erhitzt, danach wurden 118,5 g POCl₃/Toluol abdestilliert,und der Rückstand wurde mit Ethylacetat (121 ml) und Wasser (408 ml) versetzt. Der pH wurde durch Zugabe von 18 ml 30%-iger Natronlauge auf pH 1 gestellt,und die Phasen wurden getrennt. Die wässrige Phase wurde zweimal mit je 200 ml Ethylacetat extrahiert,und die vereinigten organischen Phasen wurden mit Wasser (200 ml) gewaschen, getrocknet (MgSO₄), filtriert, eingeengt und am Hochvakuum getrocknet. Es wurden 14,07 g 2-Butyl-5-chlorimidazol-4-carbaldehyd (HPLC-Gehalt 79,9%) erhalten, dies entspricht einer Ausbeute von 56%,bez. auf Pentanimidsäuremethylester.

## Patentansprüche

1. Verfahren zur Herstellung von 2-substituierten 5-Chlorimidazol-4-carbaldehyden der allgemeinen Formel 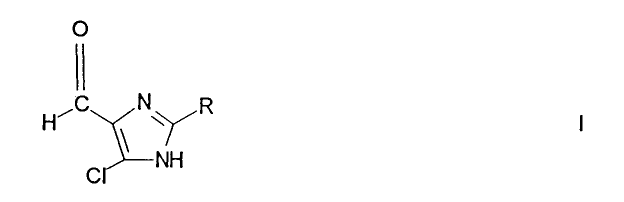 worin R Wasserstoff, eine Alkylgruppe, eine Alkenylgruppe, eine Cycloalkylgruppe oder eine an ihrem aromatischen Kern gegebenenfalls substituierte Arylgruppe oder Arylalkylgruppe bedeutet, dadurch gekennzeichnet, dass man in der ersten Stufe ein Glycinesterhydrohalogenid der allgemeinen Formel 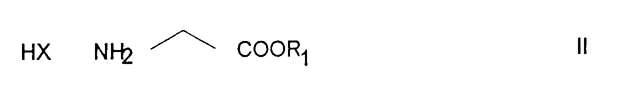 worin R₁ eine Alkylgruppe bedeutet und X für ein Halogenatom steht,mit einem Imidsäureester der allgemeinen Formel 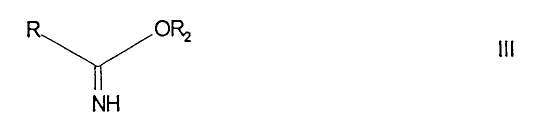 worin R die genannte Bedeutung hat und R₂ eine Alkylgruppe bedeutet,in Gegenwart einer Base zu einem 2-substituierten 3,5-Dihydroimidazol-4-on der allgemeinen Formel 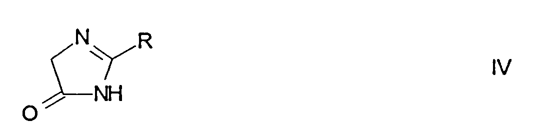 worin R die genannte Bedeutung hat, umsetzt, dieses in der zweiten Stufe mit einem N,N-substituierten Formamidacetal der allgemeinen Formel 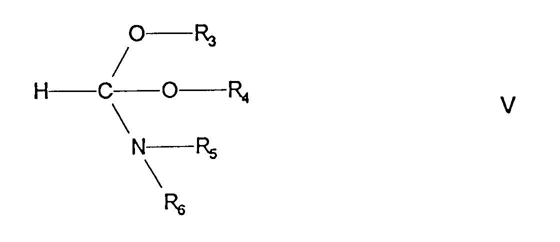 worin R₃ und R₄ gleich oder verschieden sind und eine Alkylgruppe oder eine an ihrem aromatischen Kern gegebenenfalls substituierte Arylalkylgruppe bedeuten und R₅ und R₆ gleich oder verschieden sind und für eine Alkylgruppe oder eine an ihrem aromatischen Kern gegebenenfalls substituierte Arylgruppe oder Arylalkylgruppe stehen, in ein N,N-substituiertes Aminomethylenimidazolinon der allgemeinen Formel 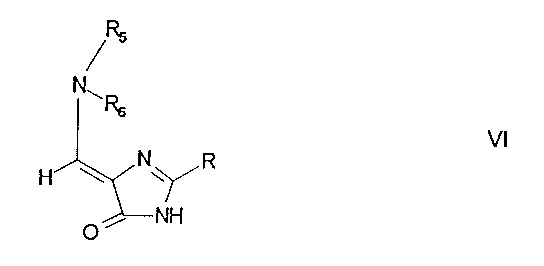 worin R, R₅ und R₆ die genannte Bedeutung haben, überführt und schliesslich in der dritten Stufe mit Phosphoroxychlorid oder Phosgen chloriert und zum Endprodukt hydrolysiert.

2. Verfahren nach Patentanspruch 1, dadurch gekennzeichnet, dass die Zwischenstufen der allgemeinen Formeln IV und VI nicht isoliert werden.

3. Verfahren nach Patentanspruch 1 oder 2, dadurch gekennzeichnet, dass als Base in der ersten Stufe ein Alkalihydroxid oder ein Alkalialkoholat verwendet wird.

4. Verfahren nach einem der Patentansprüche 1 bis 3, dadurch gekennzeichnet, dass die Umsetzungstemperatur in der ersten Stufe zwischen -20°C und 50°C liegt.

5. Verfahren nach einem der Patentansprüche 1 bis 4, dadurch gekennzeichnet, dass die Umsetzung in der zweiten Stufe,gegebenenfalls in Gegenwart eines inerten Lösungsmittels,bei einer Temperatur zwischen -50°C und 100°C erfolgt.

6. Verfahren nach einem der Patentansprüche 1 bis 5, dadurch gekennzeichnet, dass die Chlorierung mit Phosphoroxychlorid bei einer Temperatur zwischen 50°C und 150°C durchgeführt wird.

7. Verfahren nach einem der Patentansprüche 1 bis 5, dadurch gekennzeichnet, dass die Chlorierung mit Phosphoroxychlorid und N,N-Dimethylformamid oder Phosgen und N,N-Dimethylformamid bei einer Temperatur zwischen 50°C und 150°C durchgeführt wird.

8. N,N-substituierte Aminomethylenimidazolinone der allgemeinen Formel 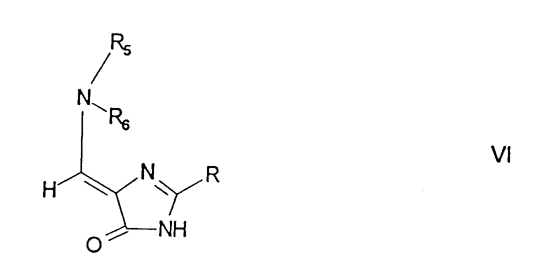 worin R, R₅ und R₆ die in Anspruch 1 genannte Bedeutung haben, in der Form des E- oder Z-Isomeren.

9. (Z)-2-Butyl-4-dimethylaminomethylen-2-imidazolin-5-on als Verbindung der allgemeinen Formel VI mit R = n-Butyl, R₅ und R₆ = Methyl gemäss Patentanspruch 8.

## Claims

1. Process for the preparation of 2-substituted 5-chloroimidazole-4-carbaldehydes of the general formula 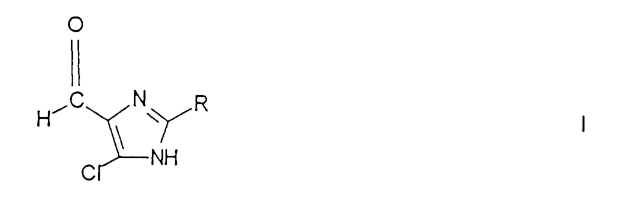 where R is hydrogen, an alkyl group, an alkenyl group, a cycloalkyl group, or an aryl group or an arylalkyl group which can be optionally substituted on the aromatic nucleus, characterized in that a glycine ester hydrohalide of the general formula 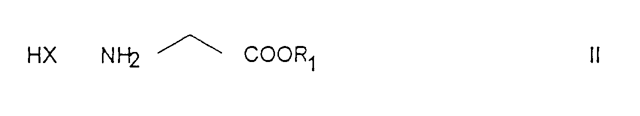 where R₁ is an alkyl group and X stands for a halogen atom, is reacted, in a first step, with an imidic acid ester of the general formula 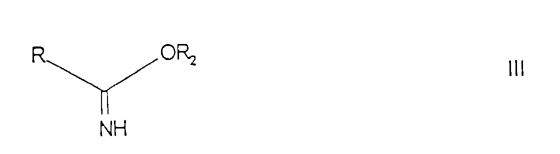 wherein R has the above-mentioned meaning and R₂ denotes an alkyl group in the presence of a base to give the 2-substituted 3,5-dihydroimidazol-4-one of the general formula 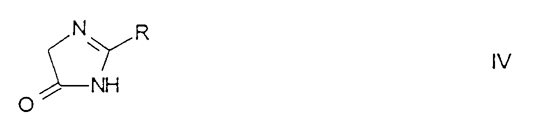 wherein R has the above-mentioned meaning, the latter one is converted, in a second step, with an N,N-substituted formamidoacetal of the general formula 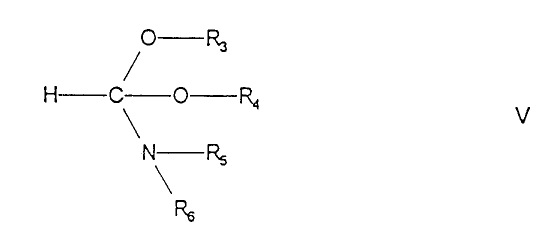 where R₃ and R₄ are the same or different and stand for an alkyl group or an arylalkyl group which can be optionally substituted on the aromatic nucleus and R₅ and R₆ are the same of different and stand for an alkyl group, or an aryl group or an arylalkyl group which can be optionally substituted on the aromatic nucleus, to give an N,N-substituted aminomethylene imidazolinone of the general formula 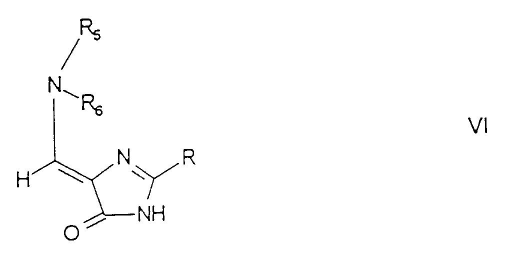 where R, R₅ and R₆ have the above-mentioned meaning and is finally chlorinated, in a third step, with phosphorus oxychloride or phosgene and hydrolyzed to give the end product.

2. Process according to claim 1, characterized in that the intermediate products of the general formulae IV and VI are not isolated.

3. Process according to claim 1 or 2, characterized in that an alkali hydroxide or alkali alcoholate is used as base in the first step.

4. Process according to one of claims 1 to 3, characterized in that the reaction temperature in the first step lies between -20°C and 50°C.

5. Process according to one of claims 1 to 4, characterized in that in the second step, the reaction is carried out at a temperature between -50°C and 100°C, optionally in the presence of an inert solvent.

6. Process according to one of claims 1 to 5, characterized in that the chlorination with phosphorus oxychloride is carried out at a temperature between 50°C and 150°C.

7. Process according to one of claims 1 to 5, characterized in that the chlorination with phosphorus oxychloride and N,N-dimethylformamide or phosgene and N,N-dimethylformamide is carried out at a temperature between 50°C and 150°C.

8. N,N-substituted aminomethylene imidazolinone of the general formula 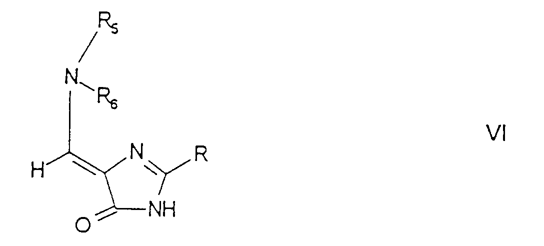 where R, R₅ and R₆ have the meanings defined in claim 1, in the form of the E- or Z-isomer.

9. (Z)-2-Butyl-4-dimethylaminomethylene-2-imidazolin-5-one, as a compound of the general formula VI according to claim 8, wherein R = n-butyl and R₅ and R₆ = methyl.

## Revendications

1. Procédé pour la préparation de 5-chloro-imidazole-4-carbaldéhydes substitués en 2 répondant à la formule générale 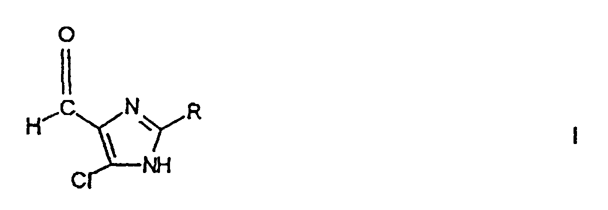 dans laquelle R signifie l'hydrogène, un groupe alkyle, un groupe alcényle, un groupe cycloalkyle ou un groupe aryle ou arylalkyle éventuellement substitué sur son noyau aromatique, caractérisé en ce que l'on fait réagir dans une première étape un halogénhydrate d'ester de glycine de formule générale 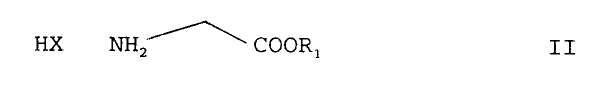 dans laquelle R₁ signifie un groupe alkyle et X représente un atome d'halogène, avec un ester d'imide acide de formule générale 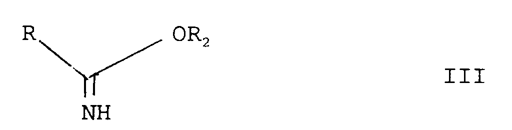 dans laquelle R possède la signification indiquée et R₂ signifie un groupe alkyle, en présence d'une base pour obtenir une 3,5-dihydroimidazole-4-one substituée en 2 de formule générale 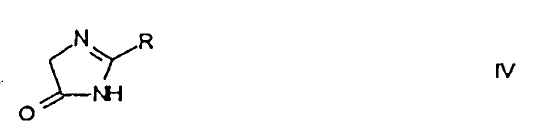 dans laquelle R possède la signification indiquée, que l'on transforme celle-ci dans la deuxième étape avec un formamide acétal N,N-substitué de formule générale 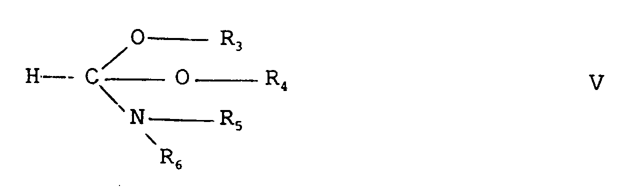 dans laquelle R₃ et R₄ sont identiques ou différents et signifient un groupe alkyle ou un groupe arylalkyle éventuellement substitué sur son noyau aromatique et R₅ et R₆ sont identiques ou différents et représentent un groupe alkyle ou un groupe aryle ou arylalkyle éventuellement substitué sur son noyau, en une aminométhylèneimidazolinone N,N-substituée de formule générale 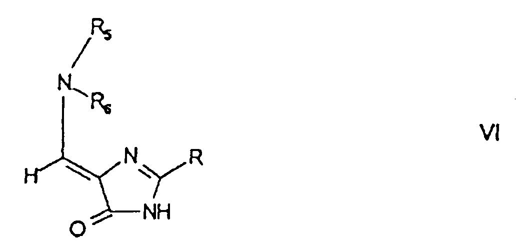 dans laquelle R, R₅ et R₆ ont la signification précédente et que, finalement, on chlore dans la troisième étape avec de l'oxychlorure de phosphore ou du phosgène et hydrolyse pour obtenir le produit final.

2. Procédé selon la revendication 1, caractérisé en ce que les stades intermédiaires des formules IV et VI ne sont pas isolés.

3. Procédé selon la revendication 1 ou 2, caractérisé en ce qu'un hydroxyde alcalin ou un alcoolate est utilisé comme base dans la première étape.

4. Procédé selon l'une des revendications 1 à 3, caractérisé en ce que la température de réaction dans la première étape se situe entre -20°C et 50°C.

5. Procédé selon l'une des revendications 1 à 4, caractérisé en ce que la réaction dans la deuxième étape a lieu, éventuellement en présence d'un solvant inerte, à une température entre -50°C et 100°C.

6. Procédé selon l'une des revendications 1 à 5, caractérisé en ce que la chloration avec de l'oxychlorure de phosphore s'effectue à une température entre 50°C et 150°C.

7. Procédé selon l'une des revendications 1 à 5, caractérisé en ce que la chloration avec de l'oxychlorure de phosphore et du N,N-diméthylformamide ou avec du phosgène et du N,N-diméthylformamide s'effectue à une température entre 50°C et 150°C.

8. Des aminométhylèneimidazolinones N,N-substituées de formule générale 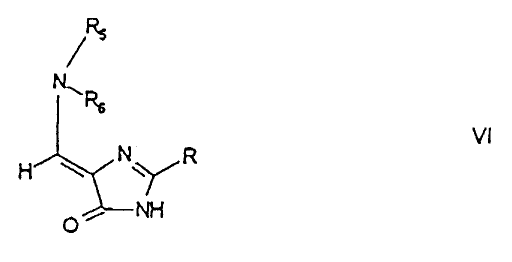 dans laquelle R, R₅ et R₆ possèdent la signification indiquée dans la revendication 1 sous la forme de l'isomère E ou Z.

9. (Z)-2-butyl-4-dimétylaminométhylène-2-imidazoline-5-one en tant que composé de formule générale VI avec R = n-butyle, R₅ et R₆ = méthyle selon la revendication 8.
